Europäisches Patentamt

European Patent Office

Office européen des brevets

(19)

(11) **EP 1 477 177 A1**

(12) **EUROPEAN PATENT APPLICATION**
published in accordance with Art. 158(3) EPC

(43) Date of publication:
**17.11.2004 Bulletin 2004/47**

(21) Application number: **03742669.9**

(22) Date of filing: **20.02.2003**

(51) Int Cl.⁷: **A61K 35/78**, A61K 9/20,
A61K 9/48, A61K 31/352,
A61K 31/37, A61K 31/56,
A61P 3/04, A61P 3/06,
A61P 3/10, A61P 9/10,
A61P 9/12, A61P 43/00,
C07D 311/18, C07D 311/58,
C07J 63/00

(86) International application number:
**PCT/JP2003/001839**

(87) International publication number:
**WO 2003/070263 (28.08.2003 Gazette 2003/35)**

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **20.02.2002 JP 2002043905
02.08.2002 JP 2002226487**

(71) Applicant: **KANEKA CORPORATION
Osaka-shi, Osaka 530-8288 (JP)**

(72) Inventors:
• **YAMASHITA, Koki
  Osaka-shi, Osaka 550-0024 (JP)**
• **ONO, Naoki,Sanseiso
  Tarumi-ku Kobe-shi, Hyogo 655-0872 (JP)**
• **KITAMURA, Shiro
  Akashi-shi, Hyogo 673-0882 (JP)**
• **UEDA, Yasuyoshi
  Himeji-shi, Hyogo 671-1227 (JP)**

(74) Representative: **VOSSIUS & PARTNER
Siebertstrasse 4
81675 München (DE)**

(54) **PROCESS FOR PRODUCING HYDROPHOBIC GLYCYRRHIZA EXTRACT WITH HIGH QUALITIES**

(57)    It is an object of the present invention to provide a method for producing a hydrophobic licorice extract which is useful for applications, such as food, food additives, functional nutritive food, food for specified health uses, dietary supplements, drink, feedstuff, drugs, and quasi-drugs, and which has satisfactory powder characteristics.

This invention relates to a method for producing a hydrophobic licorice extract comprises performing extraction from licorice with a water-soluble organic solvent, the area of the bark of the licorice occupying at least 30% of the total surface area of the licorice. This invention also relates to a method for producing a hydrophobic licorice extract comprises crystallizing an extract extracted from licorice, or an extraction residue of the licorice, with a water-soluble organic solvent in a mixed solvent comprising water and the water-soluble organic solvent, the weight ratio, water/(water-soluble organic solvent + water), being 1/3 or more.

EP 1 477 177 A1

**Description**

Technical Field

The present invention relates to a method for producing a hydrophobic licorice extract prepared with a water-soluble organic solvent, and a method for producing crystals from the hydrophobic licorice extract.

Background Art

The use of licorice started more than 2,000 years ago both in the West and in the East. Licorice is still used today as an important material for food, etc. Active ingredients contained in extracts (hydrophobic licorice extracts) extracted from licorice with organic solvents have various effects, for example, an antioxidant effect (Demizu S., et al., Chem. Pharm. Bull., 36(9), 3474(1988)), oxidase inhibition (Haramoto I., Nishinichi-hifu, 57(3), 594(1995)), ultraviolet absorption (Japanese Unexamined Patent Application Publication No. 1-157909), an anticariogenic effect (Kawai T., Nihon Kokugakkai zasshi (Journal of Japanese Stomatological Society) , 40(5), 1245(1991)), an antiphlogistic effect (Shibata S., et al., Planta Med, 57(3), 221(1991)), cyclic AMP phosphodiesterase inhibition (Kusano A., et al., Chem. Pharm. Bull., 39, 930(1991)), and an antiallergic effect (Kakegawa H., 40, 1439(1992)). In addition, examples of recently reported effects of the active ingredients include a hypoglycemic action, visceral fat reduction, TNF production inhibition, and an inhibitory effect on elevation of blood pressure (which are disclosed in WO02/47699); inhibition of arteriosclerosis (Japanese Unexamined Patent Application Publication No. 7-53393); and an antibacterial action (Japanese Unexamined Patent Application Publication No. 8-119872). Licorice is thus useful as a food for human beings as well as for livestock and pet animals, food additives, functional nutritive food, food for specified health uses, dietary supplements, drink, feedstuff, drugs, quasi-drugs, etc. It is also greatly expected that licorice will be widely used as a material for preventing lifestyle-related diseases, such as obesity, diabetes, hypertension, and hyperlipidemia (which are sometimes called "the deadly quartet"), as disclosed in WO02/47699.

Licorice extracts can be obtained by extracting licorice or water-extracted residues of licorice, e.g., residues after the extraction of a sweet ingredient (glycyrrhizin), with various solvents, and then removing the solvents by distillation.

For example, each of Japanese Unexamined Patent Application Publications Nos. 1-149706 and 3-109314 discloses an example of extraction with a hydrophobic organic solvent; Japanese Unexamined Patent Application Publication No. 2-204495 discloses an example of extraction with a solvent prepared by mixing a hydrophobic organic solvent with a small amount of water-soluble organic solvent; Japanese Unexamined Patent Application Publication No. 7-53393 discloses an example of extraction with water and/or a water-soluble organic solvent (preferably, hot water); and Japanese Unexamined Patent Application Publication No. 1-157909 discloses an example of extraction with one of a wide variety of solvents from water-soluble organic solvents to hydrophobic organic solvents.

With respect to the kinds of licorice used in the production methods described above, Japanese Unexamined Patent Application Publication No. 1-157909 discloses *Glycyrrhiza inflata BAT.* as a preferred licorice, and Japanese Unexamined Patent Application Publication No. 3-109314 describes that *Glycyrrhiza glabra L. var.* is preferably extracted with a hydrophobic organic solvent.

When the solvents are removed by distillation from the resultant liquid extracts, it has been known that the extract tends to be obtained as a powder or as a brown solid in case extracted with a hydrophobic organic solvent (Japanese Unexamined Patent Application Publications Nos. 3-109314 and 1-149706, and Japanese Examined Patent Application Publication No. 4-34528), a yellow or brown solid or paste in case extracted with a solvent prepared by mixing a hydrophobic organic solvent with a small amount of water-soluble organic solvent (Japanese Unexamined Patent Application Publication No. 2-204495), or a brown, hygroscopic amorphous substance in case extracted with water (Japanese Unexamined Patent Application Publication No. 7-53393).

As a result of research conducted by the present inventors, it has been found that although the active ingredients, which have the effects of preventing lifestyle-related diseases, e.g., hypoglycemic action and visceral fat reduction, are suitably extracted with a water-soluble organic solvent, water-soluble impurities also tend to be extracted and mixed into the resultant extract when a water-soluble organic solvent, such as ethanol, is used for extraction. It has also been found that such a tendency becomes significant if an inexpensive aqueous water-soluble organic solvent, such as hydrous ethanol, is used.

In order to use hydrophobic licorice extracts as materials for preventing lifestyle-related diseases, etc., it is important to reduce the content of water-soluble impurities, such as glycyrrhizin.

In particular, glycyrrhizin, which is used as a medicine, produces side effects, such as elevation of blood pressure, cardiomyopathy, and hydremia (which are disclosed by Harders, H. & Rausch-Stroomann, J. G., Munch. Med. Wschr. 95, 580 (1953)); hypokalemia, plasma rennin activity reduction, pseudoaldosteronism, and flaccid quadriplegia (which are disclosed by Conn, J. W., Rovner, D. R. & Cohen, E. L., J. Am. Med. Assoc. 205, 492 (1968)); and reduction of urinary aldosterone excretion, edema, headache, and drowsiness (which are disclosed by Epstein, M. T., et al., Brit. Med. J., 1, 488 (1977)). Moreover glycyrrhizin is 150 times sweeter than sucrose. Immixing of glycyrrhizin is disadvantageous when using licorice extracts as food, etc., because of the inhibition of various effects of the licorice extracts, side effects, and high sweetness level.

In order to reduce the content of water-soluble impurities, such as glycyrrhizin, in the resultant licorice extract, preferably, the water content is decreased during extraction with an organic solvent. For that purpose, it is generally important to use licorice which has been dried as much as possible, and moreover, it is important to use a water-free organic solvent.

However, as we all know, licorice is a plant. In order to obtain licorice which has been dried as much as possible, commonly used sun drying or the like is not necessarily sufficient, and use of a dryer, etc., is required. This poses a major obstacle to industrial scale production.

Even if a water-free organic solvent is used, the organic solvent inevitably contains water brought from the licorice used and from the working environment. An organic solvent with an increased water content is thereby recovered. It is difficult to directly recycle such an organic solvent. The used organic solvent must be disposed of, or in order to remove water, special and expensive purification facilities are required. In any case, this results in an increase in production cost. The problem is particularly serious when a water-soluble organic solvent is used as an extractant.

Accordingly, if it is possible to produce a high-quality licorice extract with a low water-soluble impurity content significantly simply and inexpensively, huge merits are expected.

Furthermore, it has also been found that, with respect to a liquid extract obtained by extraction with a water-soluble organic solvent, such as ethanol, even if the solvent is removed by distillation from the liquid extract, the resultant extract is not always obtained stably as a solid, which is easy to handle, and tends to be semisolid or oily (in paste form). The reason for this is supposed to be that, as disclosed in Japanese Examined Patent Application Publication No. 4-34528, in the extraction with the water-soluble organic solvent, a large amount of impurities, such as odorous components and colored components, is extracted. Watersoluble impurities are also likely to be mixed, which also makes it difficult to obtain the extract as a solid stably.

Summary of the Invention

In view of the problems described above, it is an object of the present invention to stably obtain an extract from licorice, in particular, *Glycyrrhiza uralensis* (*Glycyrrhiza uralensis Fisch. et DC;* ural kanzo (Uralian licorice)) or *Glycyrrhiza glabra* (*Glycyrrhiza glabra L.;* yo kanzo (Western licorice)), with a water-soluble organic solvent, and preferably, an extract of *Glycyrrhiza uralensis* with a water-soluble organic solvent, simply and inexpensively, as a high-quality extract with a low content of water-soluble impurities, and more preferably as a solid which is easy to handle (i.e. having satisfactory powder characteristics).

The present inventors have conducted intensive research and have found that by subjecting licorice to extraction with a water-soluble organic solvent under certain conditions, it is possible to suitably obtain a high-quality extract with a low content of water-soluble impurities, and that a licorice extract with a water-soluble organic solvent can be suitably crystallized under certain conditions to obtain a solid which has a low content of water-soluble impurities and which is easy to handle (i.e. having satisfactory powder characteristics). Thus, the present invention has been completed based upon these findings.

In a first aspect of the present invention, a method for producing a hydrophobic licorice extract comprises performing extraction from licorice with a water-soluble organic solvent, the area of the bark of the licorice occupying at least 30% of the total surface area of the licorice.

In a second aspect of the present invention, a method for producing crystals of a hydrophobic licorice extract comprises crystallizing an extract extracted from licorice with a water-soluble organic solvent in a mixed solvent comprising water and the water-soluble organic solvent, the weight ratio, water/(water-soluble organic solvent + water), being 1/3 or more. Detailed Disclosure of the Invention

The present invention will be described in detail below. , examples of licorice which may be used in the present invention include leguminous plants of the genus *Glycyrrhiza,* such as *Glycyrrhiza uralensis* (*G. uralensis Fisch. et DC;* ural kanzo (Uralian licorice)), *Glycyrrhiza inflata* (*G. inflata BAT.;* choka kanzo), *Glycyrrhiza glabra* (*Glycyrrhiza glabra L.;* yo kanzo (Western licorice)), *Glycyrrhiza glabra* (*G. glabra L. var glandu rifera Regel et Herder;* nanking kanzo (Nanking licorice)), *Glycyrrhiza echinata* (*G. echinata L.;* china kanzo (Chinese licorice)), *Glycyrrhiza pallidiflora* (*G. pallidiflora Maxim;* inu kanzo), and other plants of the same genus (*Leguminosae*). These plants are produced in Sinkiang, the Northeastern region of China, the Northwestern region of China, Mongolia, Russia, Afghanistan, Iran, Turkey, and other regions.

Among them, *G. uralensis* (*G. uralensis Fisch. et DC;* ural kanzo (Uralian licorice); produced in the Northeastern region of China, the Northwestern region of China, Mongolia, Sinkiang, etc.), and *G. glabra (Glycyrrhiza glabra L.;* yo kanzo (Western licorice); produced in Russia, Afghanistan, Iran, Turkey, etc.) are preferable. Above all, *G. uralen-*

*sis* (*G. uralensis Fisch. et DC;* ural kanzo (Uralian licorice)) having high contents of the active ingredients, which will be described below, is preferably used.

In the present invention, roots, rhizomes, or stolons of the licorice are preferably used. These are used as a finely ground product, ground product, or cut product. Alternatively, periderms of the licorice are used.

Herein, the term "finely ground product" refers to powder or pieces close to powder. The term "ground product" means filamentous, fibrous, or flocculent pieces (e.g., about 5 mm to about 10 cm in length). The term "cut product" means sliced roots, rhizomes, stolons, or the like of licorice (e.g., usually, about 1 cm to about 10 cm, preferably about 1 cm to about 5 cm, and more preferably about 2 cm to about 4 cm in length; and usually, about 3 cm or less, preferably about 2 cm or less, and more preferably about 1 cm or less in diameter, sliced in rounds). Usually, the cut product is cylindrical or substantially cylindrical. Furthermore, the term "periderm" means bark on the roots, stems, stolons, and the like (e.g., about 1 cm to about 1 m in length, and about 1 mm to about 5 mm in thickness).

The finely ground product, ground product, and cut product can be obtained using conventional devices. With respect to the finely ground product, powdered pieces or pieces close to powder can be obtained, for example, with a masscolloider type mill, or a stone mill. The ground product can be obtained, for example, with a hammer mill. The cut product can be obtained by cutting the roots, etc., of licorice at a length in the range described above with a common cutter. Furthermore, the periderm can be obtained by peeling the bark on the roots, stems, stolons, and the like.

Additionally, in licorice, the composition and amount of the active ingredients may sometimes vary depending on the kind, the land of origin, the period of harvest, etc. Therefore, preferably, the amount of the active ingredients in licorice is checked by preliminary experiments, and the licorice having a large amount of active ingredients is used.

Even when extraction is carried out with a water-soluble organic solvent, in a method in which the solvent is removed by distillation from the liquid extract, it is not always possible to stably obtain a solid extract, which is easy to handle, depending on the kind, the land of origin, the period of harvest, etc. The resultant extract may sometimes be semisolid or oily (in paste form). In accordance with the second method for producing crystals of the present invention, it is possible to obtain the licorice extract stably and efficiently as crystals which are easy to handle, regardless of the kind, the land of origin, and the period of harvest of licorice.

In the present invention, the finely ground product, ground product, cut product, or periderm of licorice may be directly subjected to extraction with a water-soluble organic solvent. Alternatively, an extraction residue obtained by removing the impurities in licorice by extraction with another solvent (e.g., water, an aqueous alkaline solution, or hexane) may be subjected to extraction with a water-soluble organic solvent.

In order to remove impurities in the licorice by extraction, for example, a method is preferably performed in which the licorice is subjected to extraction with water and/or an aqueous alkaline solution.

Examples of the aqueous alkaline solution include, but are not limited to, aqueous solutions of inorganic and organic bases, such as an aqueous sodium hydroxide solution, an aqueous sodium hydrogencarbonate solution, and an aqueous ammonia solution. The concentration of the inorganic and/or organic base is, for example, about 0.1 percent(%)by weight (w/w) to 10% by weight, and preferably 0.2 % to 5 %(w/w), although not particularly limited thereto.

When extraction is carried out with water, the extraction temperature is not particularly limited. In view of operability, the lower limit is usually 1°C, preferably 5°C, more preferably 10°C, and most preferably 15°C. The upper limit is usually 80°C, preferably 75°C, more preferably 70°C, and most preferably 65°C. The extraction can be particularly suitably performed in a temperature range of about 15°C to 65°C.

When extraction is carried out with an aqueous alkaline solution, the extraction temperature is not particularly limited. In view of operability, the lower limit is usually 1°C, preferably 5°C, more preferably 10°C, and most preferably 15°C. The upper limit is usually 45°C, preferably 40°C, more preferably 35°C, and most preferably 30°C. The extraction can be particularly suitably performed in a temperature range of about 15°C to 30°C.

The process of removing impurities by extraction may be performed by a common method and is not particularly limited. For example, using a solvent in an amount of 0.1 to 20 times by weight, and preferably 1 to 10 times by weight of the licorice, extraction is carried out preferably for 2 hours or more, and more preferably for 3 hours or more. The upper limit of the extraction period is not particularly limited. The extraction is usually carried out for about one day, but may be carried out for a longer time. The extraction may be carried out one or more times as needed. Furthermore, extraction with water and extraction with an aqueous alkaline solution may be combined if necessary.

The pressure during the extraction (removal of impurities by extraction) is not particularly limited. Normal pressure or higher pressure (1 to several atm) may be employed. Alternatively, reduced pressure may be employed as required. The extraction may be carried out under reflux and under slightly pressurized conditions. xtraction (removal of impurities by extraction), the extracted liquid and residue are separated by a common separation method (e.g., pressure filtration, filtration under reduced pressure, filter pressing, centrifugation, or sedimentation). Washing is then performed with water or the like, as required, (preferably, after extraction with an aqueous alkaline solution, the alkaline component is removed, for example, by neutralization and/or washing with water). The extraction residue is thereby obtained. Additionally, in the separation process described above, a filter aid, an adsorbent, and other commonly used additives, such as activated carbon and activated clay, may be used as necessary.

The resulting extraction residue of licorice is subjected to extraction with a water-soluble organic solvent directly or after the solvent used is entirely or partially removed by a common drying method (e.g., ventilation drying, stir drying, mix drying, fluidized-bed drying, flash drying, or spray drying). In order to minimize the immixing of water-soluble impurities, such as glycyrrhizin, into the extract from licorice with a water-soluble organic solvent, preferably, water in the extraction residue is removed as much as possible.

In the present invention, a water-soluble organic solvent is used as the solvent, which allows active ingredients to be suitably extracted from the licorice or the extraction residue of licorice.

Examples of the water-soluble organic solvent which can be suitably used for the extraction include, but are not limited to, monohydric alcohols having 1 to 4 carbon atoms, such as methanol, ethanol, propanol, and butanol; acetone; and mixtures thereof. Among them, preferred are monohydric alcohols having 2 to 4 carbon atoms, acetone, and mixtures thereof. More preferred are ethanol, acetone, and mixtures thereof. In view of safety to the human body, ethanol is particularly preferable as the solvent. From the standpoint that the immixing of glycyrrhizin is minimized, which will be described below, preferred are monohydric alcohols having 1 to 3 carbon atoms, acetone, and mixtures thereof; more preferred are monohydric alcohols having 2 to 3 carbon atoms, acetone, and mixtures thereof; still more preferred are ethanol, acetone, and mixtures thereof; and particularly preferred is acetone.

These water-soluble organic solvents can be used suitably in a dehydrated state. In view of recovery and recycling, a mixed solvent comprising the water-soluble organic solvent and water may be used. In such a case, from the general standpoint of efficient extraction of active ingredients, filterability of the residue after extraction, etc., the water content is usually 50 percent by volume(v/v) or less, preferably 40 %(v/v) or less, more preferably 30 %(v/v) or less, still more preferably 20 % (v/v) or less, and particularly preferably 10 %(v/v) or less. In order to minimize the immixing of water-soluble impurities, such as glycyrrhizin, a further decrease in the water content is preferred, i.e., usually 8 %(v/v) or less, preferably 4 %(v/v) or less, and more preferably 2 %(v/v) or less. The lower limit is preferably 0.5 %(v/v), although not particularly restricted.

It should be noted that the water content described above is the content of the extraction system. Of course, other solvents may also be present in an amount not causing adverse effects.

Any extraction temperature may be set between the solidifying temperature and the boiling temperature of the system. The lower limit is usually 1°C, preferably 5°C, more preferably 10°C, and most preferably 15°C. The upper limit is usually 80°C, preferably 70°C, more preferably 60°C, and most preferably 45°C. In general, the extraction can be suitably performed preferably in a range of about 1°C to 80°C, more preferably in a range of about 10°C to 60°C, and most preferably in a range of about 15°C to 45°C.

The extraction with the water-soluble organic solvent may be performed by any common method. For example, using the water-soluble organic solvent in an amount of 1 to 20 times by weight, and preferably 2 to 10 times by weight of the licorice or the extraction residue, extraction is carried out for 0.1 hours or more, preferably 0.2 hours or more, and more preferably 0.5 hours or more. Usually, each extraction operation may be suitably performed for an extraction time of about 1 to 10 hours. The upper limit of the extraction time is not particularly limited. The extraction is usually carried out for about one day, but may be carried out for a longer time. The extraction may be carried out one or more times as needed. Furthermore, two or more solvents may be used in combination if necessary.

The pressure during the extraction is not particularly limited. Normal pressure or higher pressure (1 to several atm) may be employed. Alternatively, reduced pressure may be employed as required. The extraction may be carried out under reflux and under slightly pressurized conditions.

As described above, when the alkaline component is present in excess, substantially all or all of the alkaline component is removed by neutralization or the like, and the extraction is suitably carried out in an acidic to weakly alkalinecondition, and preferably in an acidic to neutralcondition.

By the method described above, it is possible to suitably obtain an extract from licorice with a water-soluble organic solvent. As described above, it is extremely important to reduce the content of water-soluble impurities, such as glycyrrhizin, in the production of a hydrophobic licorice extract, in particular, a hydrophobic licorice extract used as a material for preventing adult diseases. Accordingly, the present inventors have further conducted intensive research on a suitable extraction method, and have found that when licorice having a high ratio of the area of the bark to the total surface area is used, even if extraction is carried out with an aqueous water-soluble organic solvent, it is possible to obtain a high-quality licorice extract with a low content of water-soluble impurities, such as glycyrrhizin.

Herein, the term "bark" means an outer tissue layer at the surface of licorice.

The ratio of the area of the bark to the total surface area is usually 30% or more, preferably 50% or more, more preferably 70% or more, still more preferably 80% or more, and particularly preferably 90% or more. With respect to the form of the licorice subjected to extraction, preferably, a cut product or periderm (including pieces mainly composed of the periderm) is used. More preferably, a cut product is used for ease of processing. In the case of the cut product, the ratio of the area of the bark to the total surface area is preferably 50% or more, more preferably 70% or more, still more preferably 80% or more, and particularly preferably 90% or more. The ratio of the area of the bark can be calculated, for example, with respect to the cut product, on the assumption that each piece is cylindrical.

For extraction of the licorice in the form described above, the water-soluble organic solvent described above can be suitably used. Particularly, preferred are monohydric alcohols having 2 to 4 carbon atoms, acetone, and mixtures thereof. More preferred are monohydric alcohols having 2 to 3 carbon atoms, acetone, and mixtures thereof. Still more preferred are ethanol, acetone, and mixtures thereof. In particular, when ethanol is used, the maximum effect is achieved. As the water-soluble organic solvent, in addition to a water-free solvent, an aqueous solvent can also be used suitably. In case of aqueous solvent, the water content is usually about 30 %(v/v) or less, preferably about 20 %(v/v) or less, more preferably about 10 %(v/v) or less, and still more preferably about 8 %(v/v) or less. The lower limit is not particularly limited. From a practical point of view, the water content is usually about 3 %(v/v) or more, and preferably about 4 %(v/v) or more.

In accordance with the method described above, it is possible to suitably obtain a high-quality extract with a low content of water-soluble impurities. The glycyrrhizin content in the resultant extract can be minimized to preferably 0.001 to 0.5 %(w/w). Even when an inexpensive aqueous water-soluble organic solvent is used as an extractant without specially drying the licorice, it is also possible to suitably obtain a high-quality extract.

In view of the stability of active ingredients, extraction with a water-soluble organic solvent is preferably carried out in a deoxygenated atmosphere, such as in an inert gas atmosphere using nitrogen gas or the like.

Next, the second method of the present invention for producing a solid, which is easy to handle, by crystallizing the extract thus obtained from licorice with the water-soluble organic solvent will be described below.

In the second method of the present invention, with respect to an extract extracted from licorice with a water-soluble organic solvent, the solvent is removed by a common solvent-removal method (e.g., atmospheric concentration, vacuum concentration, freeze drying, or freeze concentration), and then the extract is crystallized in a mixed solvent comprising water and a water-soluble organic solvent, the weight ratio, water/(water-soluble organic solvent + water), being 1/3 or more, the water being used as a poor solvent.

As the extract to be crystallized, the high-quality extract from licorice with a high ratio of the area of the bark to the total surface area is most suitable, although not limited thereto.

Examples of the water-soluble organic solvent used for crystallization include, but are not limited to, monohydric alcohols having 1 to 4 carbon atoms, such as methanol, ethanol, propanol, and butanol; acetone; and mixtures thereof. Among them, preferred are monohydric alcohols having 2 to 3 carbon atoms, acetone, and mixtures thereof. More preferred are ethanol, acetone, and mixtures thereof. In view of safety to the human body, ethanol is particularly preferable as the solvent.

Alternatively, the crystallization process may be preferably performed after removing impurities by adsorption with activated carbon or a resin (e.g., an ion exchange resin or synthetic adsorbent resin) and/or fractionation. From the standpoint that the glycyrrhizin content in the crystals is reduced, more preferably, the crystallization process is performed after removing water-soluble impurities, such as glycyrrhizin, by adsorption and/or fractionation.

In the crystallization process of the present invention, the weight ratio, water/(water-soluble organic solvent + water) is usually 1/3 or more, preferably 1/2 or more, more preferably 2/3 or more, still more preferably 3/4 or more, and particularly preferably 9/10 or more. In view of the acceleration of crystallization and improvement in recovery rate, the weight ratio is preferably higher, and the upper limit of the weight ratio, water/(water-soluble organic solvent + water) is 1. The ratio may be slightly changed depending on the quality, etc., of the extract extracted from licorice with the water-soluble organic solvent. Of course, other solvents may also be present in an amount not causing adverse effects.

The crystallization process of the present invention may be performed by decreasing the solubility of licorice extract extracted with the water-soluble organic solvent in the mixed solvent having the weight ratio described above, or by changing the water-soluble organic solvent in the licorice extract solution to a solvent having the weight ratio described above to decrease the amount of the dissolved licorice extract.

As a specific crystallization method, a common crystallization process may be used. Examples of the crystallization process include crystallization by concentration (evaporation), a crystallization process by solvent replacement (such as a process in which the solution containing the water-soluble organic solvent is subjected to replacement so that the weight ratio described above is satisfied), and a crystallization process using a poor solvent (such as a process in which an extract extracted from licorice with a water-soluble organic solvent is added into water). These processes may be combined, or other crystallization processes may also be combined with these processes as required. During the crystallization, a seed crystal may be added as necessary.

As a preferred embodiment of the present invention, the crystallization process using the poor solvent will be described in detail below.

In this process, preferably, a solution of the extract extracted from licorice with the water-soluble organic solvent (liquid extract or its concentrate) is added to water. In view of filterability, etc., the adding time is preferably 1/2 hours or more, more preferably 1 hour or more, and most preferably 2 hours or more.

From the standpoint that crystals which are easy to handle (i.e., which have satisfactory powder characteristics) are obtained, the crystallization temperature is usually 50°C or less, preferably 35°C or less, more preferably 20°C or less, still more preferably 10°C or less, and particularly preferably 5°C or less. The lower limit is the solidifying temperature of the system.

In the crystallization process described above, if dispersion is low, the properties of the resultant crystals tend to be degraded. Therefore, crystallization is preferably performed under stirring or under flow. The extent of stirring or flow is not particularly limited. The power requirement for stirring per unit volume is, for example, 0.1 kW/m$^3$ or more, preferably 0.2 kW/m$^3$ or more, and more preferably 0.3 kW/m$^3$ or more.

In view of economy, etc., the crystallization concentration (the dry weight of the extract relative to the solvent weight) at the completion of crystallization, is usually 1 %(w/w) or more, preferably 2 %(w/w) or more, and more preferably 3 %(w/w) or more.

After the crystallization, the coexisting solvent may be subsequently removed by a common solvent-removal method (e.g., atmospheric concentration, vacuum concentration, freeze drying, freeze concentration, or spray drying). Alternatively, after the crystals are obtained by separating slurry by a common solid-liquid separation method, such as pressure filtration, filtration under reduced pressure, or centrifugation, drying may be performed by a common drying method (e.g., ventilation drying under atmospheric or reduced pressure, stir drying, mix drying, fluidized-bed drying, or flash drying).

In the latter method, i.e., in the method in which drying is performed after the solid-liquid separation, since the water-soluble impurities can be appropriately removed into the mother liquor, an extract with a high content of active ingredients can be obtained.

The crystals of the hydrophobic licorice extract obtained after drying are generally a yellowish brown to blackish brown solid or powder.

Additionally, in the production method of the present invention, in view of the stability of the active ingredients, the series of processes, in particular, the processes such as extraction with the water-soluble organic solvent and/or crystallization are preferably performed in a deoxygenated atmosphere, such as in an inert gas atmosphere using nitrogen gas or the like. In order to inhibit oxidation, the processes, such as extraction with the water-soluble organic solvent and/or crystallization, can be performed in the presence of an antioxidant, such as ascorbic acid, sodium ascorbate, ascorbyl palmitate, ascorbyl stearate, or tocopherol.

The weight ratio of the crystals of the hydrophobic licorice extract thus obtained to the licorice (on a dry basis) is usually 0.05 or more, and the active ingredients are efficiently recovered. On the other hand, the glycyrrhizin content in the dry extract, on a dry basis, is low usually at 0.5 %(w/w) or less, preferably at 0.3 %(w/w) or less, more preferably at 0.2 %(w/w) or less, and most preferably at 0.1 %(w/w) or less.

Examples of active ingredients contained in the hydrophobic licorice extract crystals obtained according to the present invention include glycycoumarin and glycyrin, which are 3-arylcoumarin derivatives; dehydroglyasperin C and dehydroglyasperin D, which are isoflav-3-ene derivatives; glyasperin D, which is an isoflavan derivative; glyasperin B and glycyrrhisoflavanone, which are isoflavanone derivatives; glyurallin B, semilicoisoflavone B, and glycyrrhisoflavone, which are isoflavone derivatives; and isoliquiritigenin, which is a chalcone derivative.

The total amount of the active ingredients described above, on a dry basis, is usually 5 % (w/w) or more, preferably 6 % (w/w) or more, and more preferably 7 %(w/w) or more, (usually the maximum being 50 %(w/w) or less), relative to the hydrophobic licorice extract crystals obtained according to the present invention. Thus, the active ingredients are efficiently recovered. In particular, if the hydrophobic licorice extract crystals are obtained by the crystallization method using the poor solvent, the total amount of the active ingredients on a dry basis can be increased.

For example, preferably, the hydrophobic licorice extract crystals contain at least one ingredient selected from the group consisting of glycycoumarin, glycyrin, dehydroglyasperin C, dehydroglyasperin D, glyasperin B, and glyasperin D, as the main ingredients, in an amount of usually 0.5 %(w/w) or more, and preferably 1 %(w/w) or more, on a dry basis. More preferably, the hydrophobic licorice extract crystals contain glycycoumarin, glycyrin, dehydroglyasperin C, dehydroglyasperin D, glyasperin B, and glyasperin D each in an amount of usually 0.5 %(w/w) or more, and preferably 1 %(w/w) or more, on a dry basis. Among various kinds of licorice, in particular, *G. uralensis* (*G. uralensis Fisch. et DC;* ural kanzo (Uralian licorice)) can provide extract crystals containing large amounts of these ingredients.

In addition, for example, preferably, the hydrophobic licorice extract crystals contain at least one ingredient selected from the group consisting of glycyrrhisoflavanone, glycyrrhisoflavone, glyurallin B, semilicoisoflavone B, and isoliquiritigenin in an amount of usually 0.01 %(w/w) or more, and preferably 0.02 %(w/w) or more, on a dry basis. More preferably, the hydrophobic licorice extract crystals contain glycyrrhisoflavanone, glycyrrhisoflavone, glyurallin B, semilicoisoflavone B, and isoliquiritigenin each in an amount of usually 0.01 %(w/w) or more, and preferably 0.02 %(w/w) or more, on a dry basis. Dehydroglyasperin D is a compound which was first reported in PCT/JP02/10572.

The active ingredients in the crystals are also peroxisome proliferator-activated receptor ligand agents, i.e., PPAR ligand activators, and are significantly effective in preventing and treating, for example, Type 2 diabetes, hyperinsulinism, lipid metabolism abnormality, obesity, hypertension, and arteriosclerosis or in preventing and treating lifestyle-related diseases (PCT/JP02/10572; A. Okuno, et al., Journal of Clinical Investigation, 101, 1354-1361, 1998; R. A. Degronze, et al., Diabetes Care, 14, 173-194, 1991).

The hydrophobic licorice extract of the present invention can be used without limitation and is particularly useful for food applications (e.g., food, functional nutritive food, food for specified health uses, dietary supplements, and drink).

The hydrophobic licorice extract obtained according to the present invention can also be formulated in tablets and capsules. Furthermore, other materials for formulations may be appropriately mixed with the hydrophobic licorice extract by a conventional method. Examples of such materials include, but are not limited to, excipients, disintegrants, lubricants, binders, antioxidants, colorants, coagulation inhibitors, absorbefacients, and stabilizers. Best Mode for Carrying Out the Invention

While the present invention will be described in more detail based on the examples below, it is to be understood that the invention is not limited thereto.

The content of glycyrrhizin and the contents of active ingredients (glycyrrhisoflavanone, isoliquiritigenin, glycycoumarin, semilicoisoflavone B, glycyrrhisoflavone, dehydroglyasperin C, glycyrin, glyasperin B, glyasperin D, dehydroglyasperin D, and glyurallin B) were determined by HPLC analysis under the following conditions.

(Analysis conditions for glycyrrhizin)

Column: J'sphere ODS-H80 (YMC)

4.6 mm (inside diameter) $\times$ 250 mm (length)

Column temperature: 40°C
Mobile phase: acetonitrile:10 mM phosphoric acid aqueous solution = 33:67 (v/v)
Flow rate: 1 ml/min
Detection wavelength: 254 nm
Retention time for glycyrrhizin: 27.1 min

(Analysis conditions for active ingredients)

Column: J'sphere ODS-H80 (YMC)

4.6 mm (inside diameter) $\times$ 250 mm (length)

Column temperature: 40°C
Mobile phase: under gradient conditions where the percentage of acetonitrile to 10 mM phosphoric acid aqueous solution was maintained at 35% until 15 minutes after the start of analysis, increased at a constant rate after 15 minutes so that the percentage was 70% after 65 minutes, and maintained at 70% from 65 minutes to 70 minutes.
Flow rate: 1 ml/min
Detection wavelength: 254 nm
Retention time for each ingredient:

13.3 min for glycyrrhisoflavanone
14.7 min for isoliquiritigenin
32.7 min for glycycoumarin
34.2 min for semilicoisoflavone B
36.3 min for glycyrrhisoflavone
37.2 min for dehydroglyasperin C
48.1 min for glycyrin
51.1 min for glyasperin B
52.7 min for glyasperin D
55.2 min for dehydroglyasperin D
58.7 min for glyurallin B

(EXAMPLE 1)

To 1.0 kg of a cut product of licorice (*Glycyrrhiza uralensis*) (the ratio of the area of the bark to the total surface area of licorice being about 80%) was added 5.0 kg of 99.5 %(v/v) ethanol, and extraction was carried out at 25°C for 5 hours. A residue was then removed by filtration to prepare a liquid extract. The solvent in the liquid extract was removed under reduced pressure, and 108.9 g of a concentrate (containing 54.5 g of a hydrophobic licorice extract) was thereby obtained.

To 360 g of water under strong stirring at 5°C ($0.3 \, kw/m^3$), 40.0 g of the concentrate (containing 20.0 g of the hydrophobic licorice extract) was added dropwise over 1.5 hours, and thereby crystals were precipitated. After subsequent stirring at 5°C for 1 hour, the resultant crystals were subjected to filtration under reduced pressure, and then washed with 40 g of water cooled to 5°C. The resultant wet crystals were dried under reduced pressure, and thereby 15.8 g of hydrophobic licorice extract crystals were obtained. The contents of glycyrrhizin and active ingredients in the hydrophobic licorice extract crystals are shown in Table 1 below.

Table 1

| Compound | Content (wt%) |
|---|---|
| Glycyrrhizin | 0.01 |
| Glycyrrhisoflavanone | 0.19 |
| Isoliquiritigenin | 0.05 |
| Glycycoumarin | 3.46 |
| Semilicoisoflavone B | 0.47 |
| Glycyrrhisoflavone | 0.75 |
| Dehydroglyasperin C | 6.19 |
| Glycyrin | 2.18 |
| Glyasperin B | 4.14 |
| Glyasperin D | 7.38 |
| Dehydroglyasperin D | 3.94 |
| Glyurallin B | 0.13 |
| Total amount of active ingredients (%) | 28.88 |

(EXAMPLE 2)

Hydrophobic licorice extract crystals (15.7 g) were obtained as in Example 1 except that 95.0 %(v/v) ethanol was used. The contents of glycyrrhizin and active ingredients in the hydrophobic licorice extract crystals are shown in Table 2 below.

Table 2

| Compound | Content (wt%) |
|---|---|
| Glycyrrhizin | 0.22 |
| Glycyrrhisoflavanone | 0.13 |
| Isoliquiritigenin | 0.03 |
| Glycycoumarin | 2.98 |
| Semilicoisoflavone B | 0.46 |
| Glycyrrhisoflavone | 0.64 |
| Dehydroglyasperin C | 7.09 |
| Glycyrin | 2.08 |
| Glyasperin B | 3.89 |
| Glyasperin D | 5.49 |
| Dehydroglyasperin D | 4.53 |
| Glyurallin B | 0.12 |
| Total amount of active ingredients (%) | 27.44 |

(EXAMPLE 3)

To 1.0 kg of a finely ground product of licorice (*Glycyrrhiza uralensis*) was added 5.0 kg of 99.5 %(v/v) ethanol, and stirring was performed at 25°C for 5 hours. A residue was then removed by filtration to prepare a liquid extract. The solvent in the liquid extract was removed under reduced pressure, and 181.8 g of a concentrate (containing 90.9 g of a hydrophobic licorice extract) was thereby obtained.

To 360 g of water under strong stirring at 5°C (0.3 kw/m$^3$), 40.0 g of the concentrate (containing 20.0 g of the hydrophobic licorice extract) was added dropwise over 1.5 hours, and thereby crystals were precipitated. After subsequent stirring at 5°C for 1 hour, the resultant crystals were subjected to filtration under reduced pressure, and then washed with 40 g of water cooled to 5°C. The resultant wet crystals were dried under reduced pressure, and thereby 16.2 g of hydrophobic licorice extract crystals was obtained. The contents of glycyrrhizin and active ingredients in the hydrophobic licorice extract crystals are shown in Table 3 below.

Table 3

| Compound | Content (wt%) |
|---|---|
| Glycyrrhizin | 0.31 |
| Glycyrrhisoflavanone | 0.16 |
| Isoliquiritigenin | 0.03 |
| Glycycoumarin | 3.67 |
| Semilicoisoflavone B | 0.51 |
| Glycyrrhisoflavone | 0.66 |
| Dehydroglyasperin C | 4.54 |
| Glycyrin | 0.81 |
| Glyasperin B | 3.68 |
| Glyasperin D | 4.68 |
| Dehydroglyasperin D | 2.35 |
| Glyurallin B | 0.09 |
| Total amount of active ingredients (%) | 21.18 |

(EXAMPLE 4)

To 1.0 kg of the finely ground product of licorice (*Glycyrrhiza uralensis*) used in Example 3 was added 10 L of water, and stirring was performed at 60°C for one day. A residue was obtained by filtration and dried under reduced pressure. To the resultant powder was added 5 kg of 99.5 %(v/v) ethanol, and extraction was carried out at 25°C for 5 hours. A residue was then removed by filtration to prepare a liquid extract. The solvent in the liquid extract was removed under reduced pressure, and 178.0 g of a concentrate (containing 89.0 g of a hydrophobic licorice extract) was thereby obtained.

To 360 g of water under strong stirring at 5°C (0.3 kw/m$^3$), 40.0 g of the concentrate (containing 20.0 g of the hydrophobic licorice extract) was added dropwise over 1.5 hours, and thereby crystals were precipitated. After subsequent stirring at 5°C for 1 hour, the resultant crystals were subjected to filtration under reduced pressure, and then washed with 40 g of water cooled to 5°C. The resultant wet crystals were dried under reduced pressure, and thereby 16.2 g of hydrophobic licorice extract crystals was obtained. The contents of glycyrrhizin and active ingredients in the hydrophobic licorice extract crystals are shown in Table 4 below.

Table 4

| Compound | Content, (wt%) |
|---|---|
| Glycyrrhizin | 0.44 |
| Glycyrrhisoflavanone | 0.04 |
| Isoliquiritigenin | 0.04 |
| Glycycoumarin | 3. 38 |
| Semilicoisoflavone B | 0.48 |
| Glycyrrhisoflavone | 0.63 |
| Dehydroglyasperin C | 3.80 |

Table 4   (continued)

| Compound | Content, (wt%) |
|---|---|
| Glycyrin | 0.80 |
| Glyasperin B | 3.99 |
| Glyasperin D | 5.14 |
| Dehydroglyasperin D | 2.66 |
| Glyurallin B | 0.12 |
| Total amount of active ingredients (%) | 21.08 |

(COMPARATIVE EXAMPLE 1)

To 1.0 kg of the finely ground product of licorice (*Glycyrrhiza uralensis*) used in Example 3 was added 5.0 kg of 99.5 %(v/v) ethanol, and stirring was performed at 25°C for 5 hours. A residue was then removed by filtration to prepare a liquid extract. The solvent in the liquid extract was removed under reduced pressure, and then drying under reduced pressure (at 40°C, in full vacuum, for one night) was performed. Thereby, 96.1 g of an oily hydrophobic licorice extract was obtained. The contents of glycyrrhizin and active ingredients in the hydrophobic licorice extract crystals are shown in Table 5 below.

Table 5

| Compound | Content (wt%) |
|---|---|
| Glycyrrhizin | 0.28 |
| Glycyrrhisoflavanone | 0.03 |
| Isoliquiritigenin | 0.03 |
| Glycycoumarin | 2.84 |
| Semilicoisoflavone B | 0.42 |
| Glycyrrhisoflavone | 0.51 |
| Dehydroglyasperin C | 2.15 |
| Glycyrin | 0.68 |
| Glyasperin B | 3.14 |
| Glyasperin D | 3.78 |
| Dehydroglyasperin D | 1.85 |
| Glyurallin B | 0.05 |
| Total amount of active ingredients (%) | 15.48 |

(EXAMPLE 5)

To 10 g of the finely ground product of licorice (*Glycyrrhiza uralensis*) used in Example 3 was added 50 g of acetone, and stirring was performed at 25°C for 5 hours. A residue was then removed by filtration to prepare a liquid extract. The solvent in the liquid extract was removed under reduced pressure, and 1.26 g of a concentrate (containing 0.63 g of a hydrophobic licorice extract) was thereby obtained.

To 11.3 g of water under strong stirring at 5°C (0.3 kw/m$^3$), 1.26 g of the concentrate (containing 0.63 g of the hydrophobic licorice extract) was added dropwise over 1.5 hours, and thereby crystals were precipitated. After subsequent stirring at 5°C for 1 hour, the resultant crystals were subjected to filtration under reduced pressure, and then washed with 1.2 g of water cooled to 5°C. The resultant wet crystals were dried under reduced pressure, and thereby 0.51 g of hydrophobic licorice extract crystals was obtained. The contents of glycyrrhizin and active ingredients in the hydrophobic licorice extract crystals are shown in Table 6 below.

Table 6

| Compound | Content (wt%) |
|---|---|
| Glycyrrhizin | 0.05 |
| Glycyrrhisoflavanone | 0.13 |

Table 6 (continued)

| Compound | Content (wt%) |
|---|---|
| Isoliquiritigenin | 0.03 |
| Glycycoumarin | 4.35 |
| Semilicoisoflavone B | 0.59 |
| Glycyrrhisoflavone | 0.75 |
| Dehydroglyasperin C | 4.81 |
| Glycyrin | 0.94 |
| Glyasperin B | 4.18 |
| Glyasperin D | 5.04 |
| Dehydroglyasperin D | 2.52 |
| Glyurallin B | 0.09 |
| Total amount of active ingredients (%) | 23.43 |

(EXAMPLES 6 to 12, COMPARATIVE EXAMPLES 2 to 3)

To 2 ml of hydrous ethanol was added 200 mg of the hydrophobic licorice extract crystals obtained in Example 1, and stirring was performed at 20°C for 1 hour. The properties of the hydrophobic licorice extract were observed. The weight ratio of water/(ethanol + water) and the properties of the hydrophobic licorice extract are shown in Table 7 below.

Table 7

|  | Weight ratio water/(ethanol + water) | Liquid properties |
|---|---|---|
| Example 6 | 0.92 | Slurry |
| Example 7 | 0.84 | Slurry |
| Example 8 | 0.75 | Slurry |
| Example 9 | 0.66 | Slurry |
| Example 10 | 0.56 | Slurry |
| Example 11 | 0.46 | Slurry |
| Example 12 | 0.35 | Slurry |
| Comparative Example 2 | 0.24 | Oily |
| Comparative Example 3 | 0.12 | Oily |
| Slurry: liquid in which solid phase is dispersed Oily: liquid in which oily phase is separated | | |

As is evident from Table 7, when the water to (ethanol + water) ratio is high, the hydrophobic licorice extract can be present as the solid phase.

(EXAMPLE 13)

The hydrophobic licorice extract crystals obtained in Example 1 were mixed with corn starch, lactose, carboxymethyl cellulose, and magnesium stearate, and an aqueous poly(vinyl pyrrolidone) solution, as a binder, was further added thereto. The resultant mixture was granulated by a conventional method. Talc was added thereto, followed by mixing and tableting. Tablets with the following composition were obtained.

| Hydrophobic licorice extract crystals | 10 parts by weight |
|---|---|
| Corn starch | 25 parts by weight |
| Lactose | 15 parts by weight |
| Carboxymethyl cellulose | 10 parts by weight |
| Magnesium stearate | 3 parts by weight |
| Poly (vinyl pyrrolidone) | 5 parts by weight |
| Talc | 10 parts by weight |

Industrial Applicability

In accordance with the present invention described above, it is possible to obtain a high-quality hydrophobic licorice extract by minimizing the immixing of water-soluble impurities, such as glycyrrhizin. It is also possible to obtain a hydrophobic licorice extract having satisfactory powder characteristics.

**Claims**

1. A method for producing a hydrophobic licorice extract comprising performing extraction from licorice with a water-soluble organic solvent, the area of the bark of the licorice occupying at least 30% of the total surface area of the licorice.

2. The method according to Claim 1, wherein a cut product or periderm of the licorice are used.

3. The method according to Claim 1, wherein a cut product of the licorice are used.

4. The method according to any one of Claims 1 to 3, wherein, in the extraction from licorice with the water-soluble organic solvent, the licorice or an extraction residue of the licorice is subjected to the extraction with the water-soluble organic solvent.

5. The method according to Claim 4, wherein the extraction residue is obtained by removing impurities from the licorice by extraction with another solvent.

6. The method according to either Claim 4 or 5, wherein the extraction residue is obtained by removing impurities from the licorice by extraction with water or an aqueous alkaline solution.

7. The method according to Claim 6, wherein the extraction residue is subjected to the extraction with the water-soluble organic solvent after water in the extraction residue is removed.

8. The method according to any one of Claims 1 to 7, wherein the water-soluble organic solvent is selected from the group consisting of monohydric alcohols having 1 to 4 carbon atoms, acetone, and mixtures thereof.

9. The method according to Claim 8, wherein the water-soluble organic solvent is selected from the group consisting of monohydric alcohols having 2 to 4 carbon atoms, acetone, and mixtures thereof.

10. The method according to either Claim 8 or 9, wherein the water-soluble organic solvent is selected from the group consisting of ethanol, acetone, and mixtures thereof.

11. The method according to any one of Claims 8 to 10, wherein the water-soluble organic solvent is ethanol.

12. The method according to any one of Claims 1 to 11, wherein the water content in the water-soluble organic solvent is 30 %(v/v) or less.

13. The method according to any one of Claims 1 to 12, wherein the extraction from the licorice or the extraction residue of the licorice is performed in a deoxygenated atmosphere.

14. The method according to any one of Claims 1 to 13, wherein the licorice is *Glycyrrhiza uralensis* or *Glycyrrhiza glabra*.

15. The method according to any one of Claims 1 to 14, wherein the licorice is *Glycyrrhiza uralensis*.

16. A method 'for producing crystals of a hydrophobic licorice extract comprising crystallizing an extract extracted from licorice with a water-soluble organic solvent in a mixed solvent comprising water and the water-soluble organic solvent, the weight ratio, water/(water-soluble organic solvent + water), being 1/3 or more.

17. The method for producing the crystals of the hydrophobic licorice extract according to Claim 16, wherein the extract extracted from licorice with the water-soluble organic solvent is crystallized in the mixed solvent comprising water and the water-soluble organic solvent, the weight ratio, water/(water-soluble organic solvent + water), being 2/3 or more.

18. The method according to either Claim 16 or 17, wherein the crystallization is performed by decreasing the solubility of licorice extract extracted with the water-soluble organic solvent in the mixed solvent having said weight ratio or by changing the water-soluble organic solvent in the licorice extract solution to a solvent having said weight ratio to decrease the amount of the dissolved licorice extract.

19. The method according to any one of Claims 16 to 18, wherein the crystallization is performed by a crystallization process by concentration, a crystallization process by solvent replacement, a crystallization process using a poor solvent, or a combination thereof.

20. The method according to Claim 19, wherein the crystallization is performed by the crystallization process using the poor solvent.

21. The method according to either Claim 19 or 20, wherein the crystallization is performed by adding the extract extracted from licorice with the water-soluble organic solvent to water.

22. The method according to any one of Claims 19 to 21, wherein the extract extracted from licorice with the water-soluble organic solvent is gradually added to water over 1/2 hours or more.

23. The method according to any one of Claims 16 to 22, wherein the crystallization is performed at 50°C or less.

24. The method according to any one of Claims 16 to 23, wherein the crystallization is performed under stirring at a stirring power per unit volume of 0.1 kW/m$^3$.

25. The method according to any one of Claims 16 to 24, wherein the concentration at the completion of the crystallization is 1 %(w/w) or more, the concentration being defined as the dry weight of the extract crystals to the solvent weight.

26. The method according to any one of Claims 16 to 25, wherein the crystallization is performed after removing impurities by adsorption and/or fractionation.

27. The method according to any one of Claims 16 to 26, wherein a seed crystal is added during the crystallization.

28. The method according to any one of Claims 16 to 27, wherein the extract extracted from licorice with the water-soluble organic solvent is prepared by subjecting the licorice or an extraction residue of the licorice to the extraction with the water-soluble organic solvent.

29. The method according to Claim 28, wherein the extraction residue is obtained by removing impurities from the licorice by extraction with another solvent.

30. The method according to either Claim 28 or 29, wherein the extraction residue is obtained by removing impurities from the licorice by extraction with water or an aqueous alkaline solution.

31. The method according to Claim 30, wherein the extraction residue is subjected to the extraction with the water-soluble organic solvent after water in the extraction residue is removed.

32. The method according to any one of Claims 16 to 31, wherein the water-soluble organic solvent is selected from the group consisting of monohydric alcohols having 1 to 4 carbon atoms, acetone, and mixtures thereof.

33. The method according to Claim 32, wherein the water-soluble organic solvent is selected from the group consisting of monohydric alcohols having 2 to 3 carbon atoms, acetone, and mixtures thereof.

34. The method according to either Claim 32 or 33, wherein the water-soluble organic solvent is selected from the group consisting of ethanol, acetone, and mixtures thereof.

35. The method according to any one of Claims 16 to 34, wherein the extraction from the licorice or the extraction residue of the licorice with the water-soluble organic solvent and/or the crystallization is performed in a deoxygenated atmosphere.

36. The method according to any one of Claims 16 to 35, wherein the licorice is either *Glycyrrhiza uralensis* or *Glycyrrhiza glabra*.

37. The method according to any one of Claims 16 to 36, wherein the licorice is *Glycyrrhiza uralensis*.

38. The method according to any one of Claims 16 to 37, wherein the extract extracted from the licorice with the water-soluble organic solvent is obtained by the method according to any one of Claims 1 to 15.

39. Crystals of the hydrophobic licorice extract produced by the method according to any one of Claims 16 to 38.

40. The crystals of the hydrophobic licorice extract according to Claim 39, wherein the crystals contain a peroxisome proliferator-activated receptor ligand agent.

41. The crystals of the hydrophobic licorice extract according to either Claim 39 or 40, wherein the crystals contain at least one of glycycoumarin, glycyrin, dehydroglyasperin C, dehydroglyasperin D, glyasperin D, glyasperin B, glycyrrhisoflavanone, glyurallin B, semilicoisoflavone B, isoliquiritigenin, and glycyrrhisoflavone in a total amount of 5 %(w/w) or more, on a dry basis.

42. The crystals of the hydrophobic licorice extract according to any one of Claims 39 to 41, wherein the crystals contain at least one ingredient selected from the group consisting of glycycoumarin, glycyrin, dehydroglyasperin C, dehydroglyasperin D, glyasperin B, and glyasperin D, in an amount of 0.5 %(w/w) or more, on a dry basis.

43. The crystals of the hydrophobic licorice extract according to any one of Claims 39 to 42, wherein the crystals contain glycycoumarin, glycyrin, dehydroglyasperin C, dehydroglyasperin D, glyasperin B, and glyasperin D, each in an amount of 0.5 %(w/w) or more, on a dry basis.

44. The crystals of the hydrophobic licorice extract according to any one of Claims 39 to 43, wherein the crystals contain at least one ingredient selected from the group consisting of glycyrrhisoflavanone, glycyrrhisoflavone, glyurallin B, semilicoisoflavone B, and isoliquiritigenin in an amount of 0.01 %(w/w) or more, on a dry basis.

45. The crystals of the hydrophobic licorice extract according to any one of Claims 39 to 44, wherein the crystals contain glycyrrhisoflavanone, glycyrrhisoflavone, glyurallin B, semilicoisoflavone B, and isoliquiritigenin, each in an amount of 0.01 %(w/w) or more, on a dry basis.

46. The crystals of the hydrophobic licorice extract according to any one of Claims 39 to 45, wherein the crystals contain glycyrrhizin in an amount of 0.5 %(w/w) or less, on a dry basis.

47. The crystals of the hydrophobic licorice extract according to any one of Claims 39 to 46, wherein the crystals are used in food applications.

48. A tablet or capsule prepared using the crystals of the hydrophobic licorice extract according to any one of Claims 39 to 46.

# EP 1 477 177 A1

<table>
<tr><td colspan="2"><strong>INTERNATIONAL SEARCH REPORT</strong></td><td>International application No.<br>PCT/JP03/01839</td></tr>
</table>

**A. CLASSIFICATION OF SUBJECT MATTER**
Int.Cl$^7$ A61K35/78, 9/20, 9/48, 31/352, 31/37, 31/56, A61P3/04, 3/06, 3/10, 9/10, 9/12, 43/00, C07D311/18, 311/58, C07J63/00

According to International Patent Classification (IPC) or to both national classification and IPC

**B. FIELDS SEARCHED**

Minimum documentation searched (classification system followed by classification symbols)
Int.Cl$^7$ A61K35/78, 9/20, 9/48, 31/352, 31/37, 31/56, A61P3/04, 3/06, 3/10, 9/10, 9/12, 43/00, C07D311/18, 311/58, C07J63/00

Documentation searched other than minimum documentation to the extent that such documents are included in the fields searched

Electronic data base consulted during the international search (name of data base and, where practicable, search terms used)
CA(STN), MEDLINE(STN), BIOSIS(STN), EMBASE(STN), JSTPLUS(JOIS), JMEDPLUS(JOIS)

**C. DOCUMENTS CONSIDERED TO BE RELEVANT**

| Category* | Citation of document, with indication, where appropriate, of the relevant passages | Relevant to claim No. |
|---|---|---|
| X<br>Y | HAYASHI, Hiroaki et al., Organ specific localization of favonoids in Glycyrrhiza glabra L., Plant Science, 1996, Vol.116, No.2, pages 233 to 238, abstract, table 1 | 1-15,41-48<br>16-40 |
| X<br>Y | Hiroshi KUWAJIMA et al., Variation of Chemical Constituents in Proccessed Licorice Roots; Quantitative Determination of Saponin and Favonoid Constitents in Bark Removed and Roasted Licorice Roots, YAKUGAKU ZASSHI, 1999, Vol.119, No.12, pp.94-955, Fig. 3, table 1, page 951 | 1-15,41-48<br>16-40 |
| P,Y | WO 03/037316 A1 (KANEKA CORP.), 05 August, 2003 (05.08.03), (Family: none) | 40 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>27 May, 2003 (27.05.03) | Date of mailing of the international search report<br>10 June, 2003 (10.06.03) |
|---|---|
| Name and mailing address of the ISA/<br>Japanese Patent Office | Authorized officer |
| Facsimile No. | Telephone No. |

Form PCT/ISA/210 (second sheet) (July 1998)

16

## INTERNATIONAL SEARCH REPORT

| International application No. |
| --- |
| PCT/JP03/01839 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | Tutomu HATANO et al., Phenolic Constituens of Licorice. II. Structures of Licopyranocoumarin, Licoarylcoumarin and Glisoflavone, and Inhitory Effects of Licoraice Phenolics on Xantine Oxidase, Chem.Pharm.Bull., 1989, Vo.37, No.11, pages 3005 to 3009, table 1 | 1-48 |
| Y | Ayumu KUASNO et al., Inhibition of Adenosine 3', 5'-Cyclic Monophoshate Phosphodiesterase by Flavonoids from Licorice Roots and 4-Arylcoumarins, Chem.Pharm.Bull., 1991, Vol.39, No.4, pages 930 to 933, table 1 | 1-48 |
| Y | Isao KITAGAWA et al., Chemical Studies of Chinese Ricorice-Roots II. Five New Flavonoid Constituents from the Roots of Glycyrrhiza aspera Pall. Collected in Xinjiang, Chem.Pharm.Bull., 1998, Vol.46, No.10, pages 1511 to 1517, Fig. 1 | 1-48 |
| Y | Tsutomu HATANO et al., Phenolic Constituents of Licorice. VIII. Structures of Glicophenoneand Glicoisoflavanone, and Effects of Licorice Phenolics on Methicillin-Resistant Staphylococcus aureus, Chem.Pharm.Bull., 2000, Vol.48, No.9, pages 1286 to 1292, table 1 | 1-48 |
| Y | Yukio HIRAGA et al., Chemical and Original Guide to Licorice Root Products, The Bulletin of the Meiji College of Pharmacy, 1997, No.27, pp.9-57 | 1-48 |
| Y | Fumiyuki KIKUCHI et al., Four New Phenolic Constituents from Licorice(Root of Glycyrrhiza sp.), Heterocycles, 1990, Vol.31, No.4, pages 629 to 636 | 1-48 |
| Y | Lu Zeng et al., Four New Prenylated Flavonoids, Glyasperins A, B, C, and D from the Roots of Glycyrrhiza aspera, Heterocycles, 1992, Vol.34, No.3, pages 575 to 587, abstract | 1-48 |
| Y | Maiko SHIBANO et al., Studies on the Index Compounds for HPLC Analysis Glycyrrhiza uralensis, Heterocycles, 1997, Vol.45, No.10, pages 2053 to 2560, abstract | 1-48 |
| Y | Toshio FUKAI et al., Flavonoids from Kirghiz Licorice(Glycyrrhiza glabra), Natural Medicine, 2001, Vol.55, No.6, page 311 | 1-48 |
| Y | Toshio FUKAI et al., Isoprenoid-substituted Flavonoids from Glycyrrhiza glabra, Phytochemistry, 1996, Vol.43, No.2, pages 531 to 532 | 1-48 |

Form PCT/ISA/210 (continuation of second sheet) (July 1998)